# EUROPEAN PATENT SPECIFICATION

(11) **EP 1 694 646 B1**
(45) Date of publication and mention of the grant of the patent: **25.04.2007**
(21) Application number: 04806275.6
(22) Date of filing: 16.12.2004
(51) Int. Cl.: C07D 213/30, C07D 417/12

(54) **PROCESS FOR THE SYNTHESIS OF PIOGLITAZONE HYDROGEN CHLORIDE**
VERFAHREN ZUR SYNTHESE VON PIOGLITAZON-WASSERSTOFFCHLORID
PROCEDE DE SYNTHESE DE CHLORURE D'HYDROGENE DE PIOGLITAZONE

(30) Priority: 19.12.2003 HU 0304096
(43) Date of publication of application: 30.08.2006
(73) Proprietor: RICHTER GEDEON VEGYESZETI GYAR RT., 1103 Budapest (HU)
(72) Inventor: FISCHER, János, H-1014 Budapest (HU); FODOR, Tamás, H-1061 Budapest (HU); LÉVAI, Sándor, H-2051 Biatorbágy (HU); PETÉNYI, Endréné, H-1138 Budapest (HU); PERÉNYI, Éva, H-1117 Budapest (HU)
(74) Representative: HOFFMANN EITLE
(86) International application number: PCT/HU2004/000118
(87) International publication number: WO 2005/058827

(56) References cited:
- EP-A- 0 257 781
- EP-A- 0 816 340
- LES, ANDRZEJ ET AL: "Optimization of the Reduction of a 5-Benzylidenethiazolidine-2,4-dione Derivative Supported by the Reaction Response Surface Analysis: Synthesis of Pioglitazone Hydrochloride" ORGANIC PROCESS RESEARCH & DEVELOPMENT , 8(2), 157-162 CODEN: OPRDFK; ISSN: 1083-6160, 2004, XP002320885

## Description

The invention relates to the synthesis of the antidiabetic pharmaceutical active ingredient: pioglitazone hydrogen chloride of formula (I): 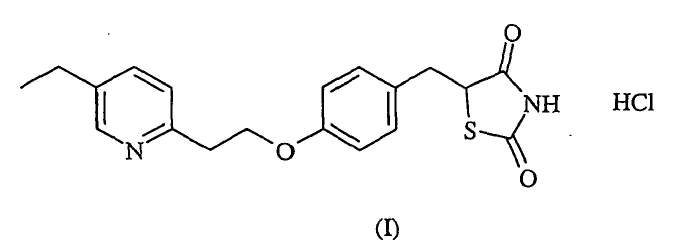 starting from 4-[2-(5-ethyl-2-pyridinyl)-ethoxy]-benzaldehyde salt of formula (II), 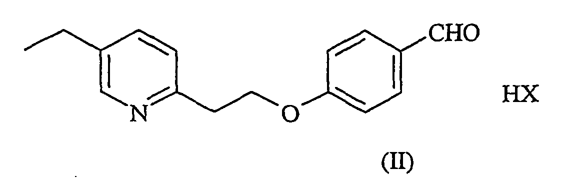 wherein HX is HCl, CF₃COOH, C₄H₄O₄, (COOH)₂
which is reacted with a base in a desired case and then reacted with thiazolidine-2,4-dione and the obtained product is treated with hydrochloric acid and the obtained 5-[[4-[2-(5-ethyl-2-pyridinyl)ethoxy]phenyl]methylen]-2,4-thiazolidinedione hydrogen chloride of formula (III) 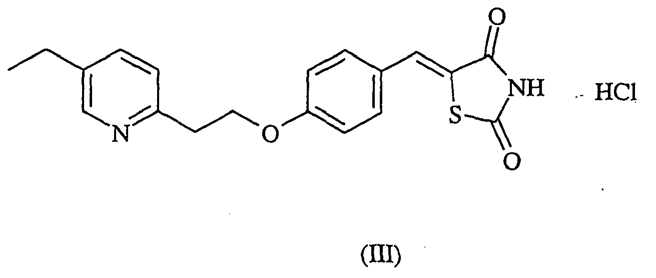 is hydrogenated in the presence of a catalyst.

Further objects of the invention are the salts of general formula (II) and the benzylidene derivative of formula (III).

Pioglitazone hydrogen chloride of formula (I) (chemical name of which is: (R,S)-5-[[4-[2-(5-ethyl-2-pyridinyl)ethoxy]phenyl]methyl]-2,4-thiazolidinedione hydrogen chloride) is described first in EP 193 256 Patent Specification.

According to this process pioglitazone hydrogen chloride is prepared from 2-imino-5-[4-[2-(5-methyl-2-pyridinyl)-ethoxy]-benzyl]-4-thiazolidinone by refluxing with 2N hydrochloric acid solution for 16 hours. The obtained product is characterized as colourless prismatic crystals. Melting point :192-193 °C.

The intermediate of formula (II) is not mentioned in this Patent Specification and this process can not be applied for scale up, because the Meerwein reaction - used for the preparation of the imino compound - has low yield and the formed by-products are environment pollutants.

EP 257781 Patent Specification describes different synthetic processes. The intermediate of 4-[2-(5-ethyl-2-pyridinyl)-ethoxy]-benzaldehyde is prepared by an ether formation reaction of 4-hydroxy-benzaldehyde and 5-ethyl-2-pyridinyl-ethanol alkyl or arylsulfonate in the presence of a base and a phase transfer catalyst. The mixture of water and dichloromethane is used as solvent. The produced by-products can be removed only with difficulty, and the obtained oily 4-[2-(5-ethyl-2-pyridinyl)-ethoxy]-benzaldehyde product is purified by column chromatography. According to this method the benzaldehyde component is produced in 32.5-62.0 % yield. The condensation thereof with 2, 4-thiazolodindione gives the so called: "benzylidene"-derivative (hereafter: "benzylidene" component in 57.6-78.5 % yield. Its chemical name is: 5-[[4-[2-(5-ethyl-2-pyridinyl)ethoxy]phenyl]methylen]-2,4-thiazolidinedione.

The catalytic hydrogenation is carried out in several kinds of solvents: e.g. dioxane, dimethylformamide, or acetic acid. A drawback of the reactions is the use of a huge amount of catalyst.For example 1.2 g of 5 % palladium on carbon catalyst has been used for 400 mg of "benzylidene" component. The catalytic hydrogenation is carried out in 6 hours at atmospheric pressure and the crude product is crystallized from ethanol to give the pioglitazone base in 54.2 % yield.

The yields are higher, when the medium of the catalytic hydrogenation is acetic acid. and the ratio of 5 % palladium on carbon catalyst and "benzylidene" component -amounts to 1:1. After removing the catalyst and concentrating the acetic acid, acetone is used to give the pioglitazone base in 67.7 % yield. The hydrogen chloride salt of pioglitazone is not mentioned in this Patent Specification.

According to EP 506273 Patent Specification the synthesis of 4-[2-(5-ethyl-2-pyridinyl)-ethoxy]-benzaldehyde base is carried out by reacting 5-ethyl-2-pyridine-ethanol metansulfonate derivative with the alkali or alkali earth salt of 4-hydroxy-benzaldehyde in ethanol. After removing the polar solvent from the reaction mixture an oily residue is obtained. According to the HPLC results (3 a)-c) reference examples) it contains only 70.0-75.3 % of 4-[2-(5-ethyl-2-pyridinyl)ethoxy]-benzaldehyde. In the next step the above "benzylidene" component is prepared by Knoevenagel reaction of 4-[2-(5-methyl-2-pyridinyl)ethoxy]-benzaldehyde and 2,4-thiazolidinedione in 73 % yield. 20 g of 5 % palladium on carbon catalyst is used for the catalytic hydrogenation of 30 g of "benzylidene" component.

The hydrogenation is carried out in vigorous conditions: at 110 °C temperature and at 100 kg/cm² pressure to give the pioglitazone base in 72 % yield. The preparation of hydrogen chloride salt of pioglitazone is not mentioned.

According to EP 816340 Patent Specification 4-[2-(5-ethyl-2-pyridinyl)-ethoxy]-benzaldehyde base is prepared in 62.0-79.8 % yield, but the pure product can be isolated only by column chromatography.

The "benzylidene" component is prepared in 77.0-95.0 % yield by optimalisation of the above Knoevenagel reaction. The pioglitazone base is produced from this by catalytic hydrogenation in dioxane or tetrahydrofurane in 63-73 % yield.

From the obtained pioglitazone base the pioglitazone HCl is prepared reacting by hydrochloric acid in ethanol and after recrystallization the crystalline pioglitazone HCl is given in 80-85 % yield.

The yield calculated for the aldehyde component is 38.8-58.9 %.

According to WO 93/13095 PCT Patent Application the reduction of the "benzylidene" component is carried out by sodium borohydride in the presence of cobalt (II) chloride catalyst. The catalyst is highly toxic, inhibits the production of red blood cells.

The purity of the product is not appropriate (only 97.7 %) and after the reduction step-as above - a special step is required for preparing the hydrogen chloride salt of pioglitazone, whose yield is 85 %.

According to WO 02/064577 PCT Patent Application the reduction of the "benzylidene" component is carried out in approx. tenfold volume of 95-97 % formic acid by catalytic hydrogenation. The conditions of the hydrogenation are milder: 75-80 °C and 8 atm.

The yield of the reduction is 97.4 %, but the quality of the product is not characterized and the pioglitazone hydrogen chloride salt, the pharmaceutical active ingredient itself is not prepared.

According to the process described in WO 03/053367 PCT Patent Application the catalytic reduction of the "benzylidene" component is carried out also in formic acid and only in 84 % yield. The preparation of the pharmaceutical active ingredient, the pioglitazone hydrogen chloride salt also is not mentioned.

The common feature of the prior art processes is that the 4-[2-(5-ethyl-2-pyridinyl)-ethoxy]-benzaldehyde intermediate and the "benzylidene" component (last intermediate) are used also as a base.

The purity of the pharmaceutical active ingredient is highly determined by the purity of the intermediates, mostly the purity of the last intermediate.

The purity of the last intermediate: "benzylidene" component is not described in the prior art.

The object of the invention is to elaborate a process to give the product via novel intermediates, by simple technology with pharmaceutically high purity and in good yield.

Surprisingly it was found by our experiments that preparing the rapidly and well crystallizing 4-[2-(5-ethyl-2-pyridinyl)-ethoxy]-benzaldehyde-oxalate salt ("benzaldehyde-oxalate"), a pure and easy to handle intermediate can be obtained, which is highly appropriate for preparing the pioglitazone hydrogen chloride pharmaceutical active ingredient with high purity.
In desired case the obtained "benzaldehyde-oxalate" is treated with a base and then condensed with thiazolidine-2,4-dione and hydrochloric acid is added to give the hydrochloride salt of the "benzylidene" component.

By catalytic hydrogenation of the obtained "benzylidene" component the pioglitazone hydrogenchloride pharmaceutical active ingredient can be prepared in one step with high purity.

The object of our invention is to elaborate a process to prepare the 5-[[4-[2-(5-ethyl-2-pyridinyl)ethoxy]phenyl]methylene]-2,4-thiazolidinedione hydrogen chloride salt (hereafter: "benzylidene hydrogen chloride") from 4-[2-(5-ethyl-2-pyridinyl)-ethoxy]-benzaldehyde-oxalate salt intermediate, then its catalytic hydrogenation afforded pioglitazone hydrogencholride in one step, with high purity and yield.

Further objects of the invention are the novel intermediates: 4-[2-(5-ethyl-2-pyridinyl)-ethoxy]-benzaldehyde salt of formula (II), preferably the oxalate salt and the 5-[[4-[2-(5-ethyl-2-pyridinyl)ethoxy]phenyl]methylene]-2,4-tiazolidinedione hydrogen chloride of formula (III).
The reaction scheme of the process according to the invention is:

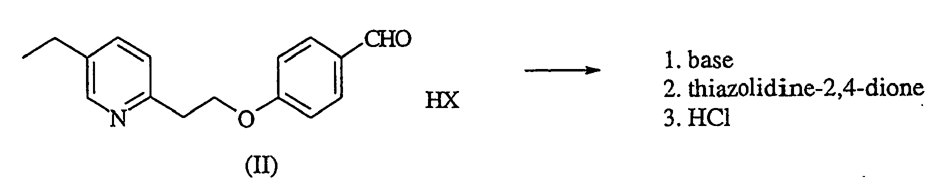

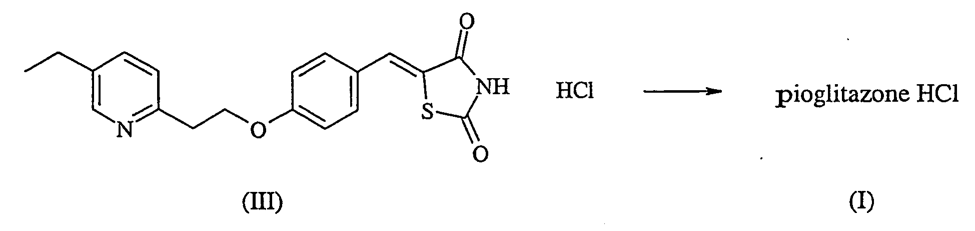

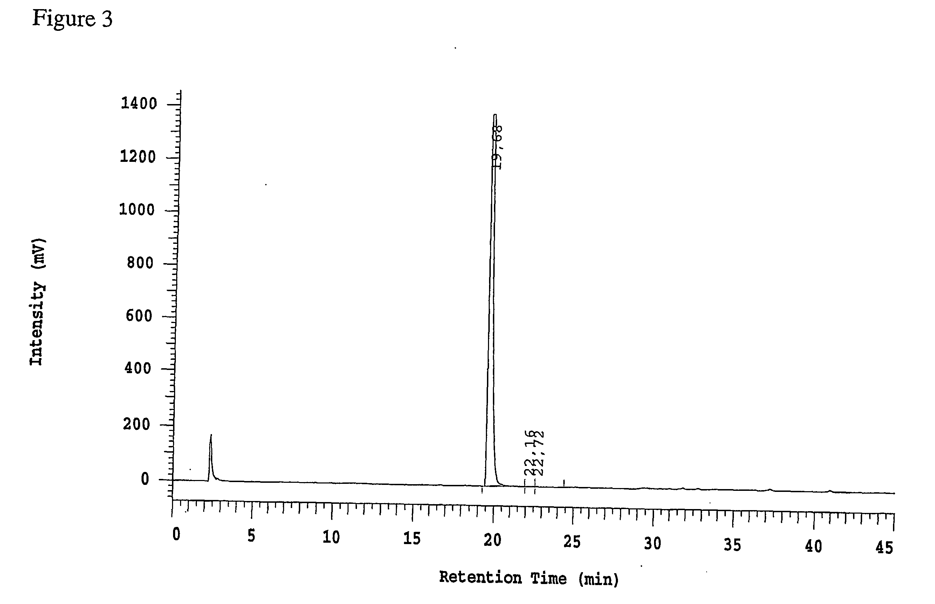

In the first step of the process according to the invention from the crude product of 4-[2-(5-ethyl-2-pyridinyl)ethoxy]benzaldehyde - obtained from the reaction of 5-ethyl-2-(2-mesyloxi-ethyl)-pyridine and 4-hydroxy-benzaldehyde - in an inert organic solvent (e.g. diethyl ether, ethyl acetate, acetone) reacting by different acids the salts (e.g. hydrogen chloride, trifluoracetate, maleate, oxalate) of 4-[2-(5-ethyl-2-pyridinyl)ethoxy]benzaldehyde of general formula (II) can be prepared (figure 3).

In desired case from the salt of formula (II) after liberating the base with 2,4-thiazolidinedione in an inert organic solvent, the 5-{[4-[2-(5-ethyl-2-pyridinyl)ethoxy]phenyl]methylene}-thiazolidine-2,4-dione can be prepared.

The hydrochloride salt of formula (III) is isolated by adding hydrochloric acid to the reaction mixture, which is also a purification process and according to this method the 5-[[4-[2-(5-ethyl-2-pyridinyl)ethoxy]phenyl]methylene]-2,4-thiazolidinedione hydrogen chloride can be obtained in 73 % yield.

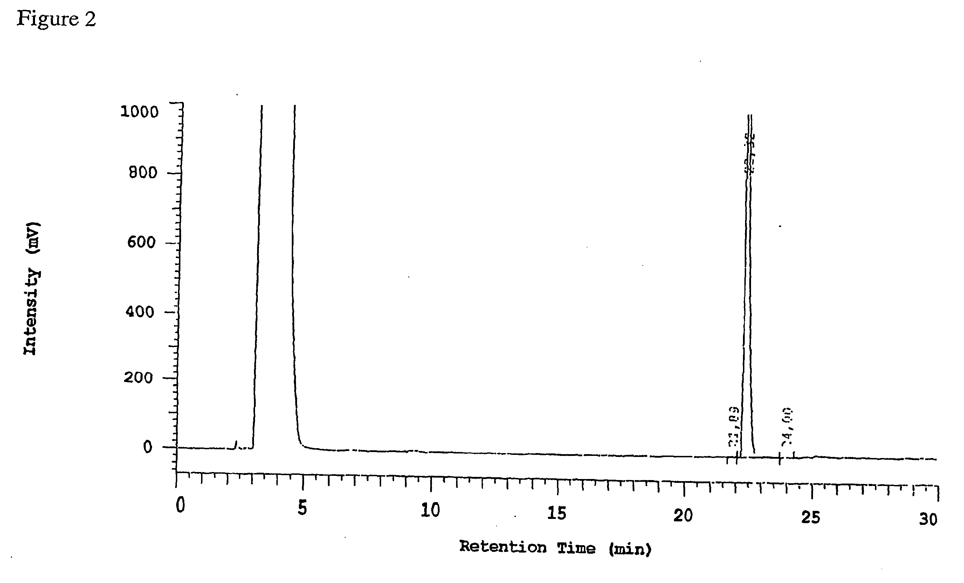

According to the HPLC studies (figure 2) the purity thereof is 99.7 % or higher, so it is a highly appropriate last intermediate for the production of a pharmaceutical active ingredient.

From the "benzylidene hydrogen chloride" of formula (III) pioglitazone hydrogen chloride can be prepared in one step by catalytic hydrogenation, preferably by hydrogenation in the presence of palladium on carbon catalyst, in an inert organic solvent, preferably in aqueous methanol under mild heating, preferably at 50-60 °C, under pressure, preferably at 5-6 bar.

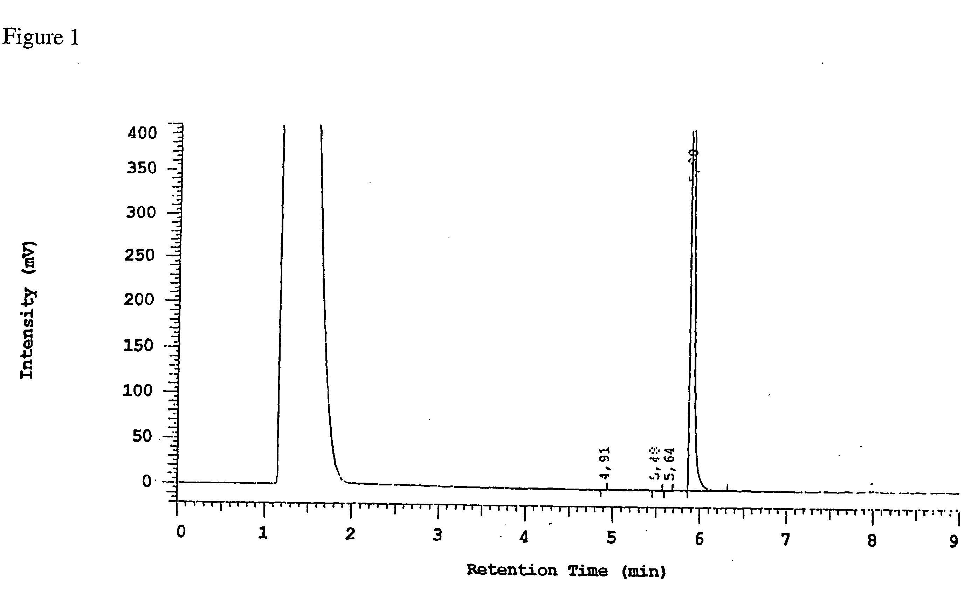

The pioglitazone hydrogen chloride can be obtained in 84 % yield, and the purity thereof is higher then 99.7 % (figure 1).

Further advantage of our process is that it is not necessary to use the relatively huge amount of costly, heterogenic catalyst.

According to our experiments 10-15 % of 10 % palladium on carbon catalyst, calculated to the "benzylidene-hydrogen chloride" is enough for the hydrogenation.

The medium of the reaction is neutral, so the use of the corrosive formic acid - mentioned in the above two prior art processes - can be avoided.

### Figures:

Figures 1-3 show the HPLC chomatograms of benzylidene" component, "benzaldehyde oxalate" and pioglitazone hydrochloride product.
Figure 1: HPLC chromatogram of pioglitazone hydrochloride
Figure 2: HPLC chromatogram of benzylidene" component
Figure 3: HPLC chromatogram of "benzaldehyde oxalate"

### HPLC method:

Column: for pioglitazone hydrochloride: Chromolit RP18e 100x4.6,
for benzylidene" component and "benzaldehyde oxalate" Lichrospher RP8 5µ 250x4 mm

| | | |
|---|---|---|
| Eluent A: | 0.03 mole / 1 KH₂PO₄ | pH=2.2 |
| Eluent B: | | Acetonitrile |

### Gradient:

| pioglitazone hydrocloride: | | | | "benzylidene"component and "benzaldehyde oxalate | | | |
|---|---|---|---|---|---|---|---|
| Time (min) | A (%) | B (%) | Flow rate (ml/min) | Time (min) | A (%) | B (%) | Flow rate (ml/min) |
| 0 | 100 | 0 | 3 | 0 | 100 | 0 | 1 |
| 10 | 30 | 70 | 3 | 30 | 30 | 70 | 1 |
| 15 | 30 | 70 | 3 | 60 | 30 | 70 | 1 |

Detection: 215 nm
Temperature: 30 °C

| | |
|---|---|
| Preparation of the samples: | the samples are solved in acetic acid |
| Retention times: | pioglitazone hydrochloride: 5.88 min. |
| | "benzylidene" component: 6.26 min. |
| | "benzaldehyde oxalate": 19.68 min |

According to figure 1 the purity of pioglitazone hydrochloride calculated by area% is 99.853 %. The "benzylidene" component is under the detection limit.

According to figure 2 the purity of "benzylidene" component calculated by area% is 99.881 %.

According to figure 3 the purity of "benzaldehyde oxalate" calculated by area% is 99.875 %.

The invention is illustrated by the following Examples:

### Example 1

The synthesis of 4-[2-(5-ethyl-2-pyridinyl)ethoxy] benzaldehyde salt

### a) The synthesis of 4-[2-(5-ethyl-2-pyridinyl]ethoxy]-benzaldehyde hydrochloride salt

15.1 g (0.1 mole) of 5-ethyl-2-(2-hydroxy-ethyl)-pyridine is solved in 150 ml of toluene and 16.8 ml (0,12 mole) of triethyl-amine is added. The solution is cooled to 0-5 °C and 11.84 g (0.1 mole) of methanesulfonyl chloride is added dropwise in 30 minutes. The reaction mixture is stirred for 4 hours at 20 °C and then it is extracted with 100 ml of water. The aqueous phase is extracted with 20 ml of toluene the unified toluene solution is dried over anhydrous Na₂SO₄.

To the filtered toluene solution 70 ml of ethanol, 17.3 g (0.14 mole) of 4-hydroxy-benzaldehyde and 15 g (0.14 mole) of K₂CO₃ are added, and it is stirred in inert atmosphere at 75-78 °C for 5 hours. After cooling to 20 °C it is extracted with 2 x 100 ml of water. The aqueous phase is extracted with 30 ml of toluene, the unified toluene solution is extracted with 2 x 50 ml of 0.5 N NaOH-solution and with 50 ml of water, dried over anhydrous Na₂SO₄, and stirred with activated carbon. The toluene is evaporated in vacuo, the residue is solved in 80 ml of ethyl acetate and under ice cooling 25 ml of 4 N hydrochloric acid / ethyl acetate is added dropwise.

The precipitated salt is stirred for 2 hours, filtered, washed with ethyl acetate and dried to give 15.1 g (52 %) of the title compound.
Melting point is 72-74 °C, HPLC purity is 96 %.

### b) The synthesis of 4-[2-(5-ethyl-2-pyridinyl]ethoxy]-benzaldehyde-trifluoracetate

15.1 g (0.1 mole) of 5-ethyl-2-(2-hydroxy-ethyl)-pyridine is solved in 150 ml of toluene and 16.8 ml (0.12 mole) of triethyl-amine is added. The obtained solution is cooled to 0-5 °C and 11.84 g (0.1 mole) of methanesulfonyl chloride is added dropwise in 30 minutes. The reaction mixture is stirred at 20 °C for 4 hours and then it is extracted with 100 ml of water. The aqueous phase is extracted with 30 ml of toluene, the unified toluene solution is extracted with 2 x 50 ml of 0.5 N NaOH-solution and with 50 ml of water, dried over anhydrous Na₂SO₄, and stirred with activated carbon. The toluene is evaporated in vacuo, the residue is solved in 80 ml of diethyl ether and under ice cooling 7.4 ml (0.1 mole) of trifluor acetic acid is added dropwise.
The precipitated salt is stirred for 2 hours, filtered, washed with ethyl ether and dried to give 21.3 g (57.7 %) of the title compound.
Melting point is 48-49 °C, HPLC purity is 98 %.

### c) The synthesis of 4-[2-(5-ethyl-2-pyridinyl]ethoxy]-benzaldehyde-maleate

15.1 g (0.1 mole) of 5-ethyl-2-(2-hydroxy-ethyl)-pyridine is solved in 150 ml of toluene and 16.8 ml (0.12 mole) of triethyl-amine is added. The obtained solution is cooled to 0-5 °C and 11.84 g (0.1 mole) of methanesulfonyl chloride is added dropwise during 30 minutes. The reaction mixture is stirred at 20 °C for 4 hours and then it is extracted with 100 ml of water. The aqueous phase is extracted with 20 ml of toluene and the unified toluene solution is dried over anhydrous Na₂SO₄.
To the filtered solution 70 ml of ethanol, 17.3 g (0.14 mole) of 4-hydroxy-benzaldehyde, 15 g (0.14 mole) of K₂CO₃ is added and stirred at 75-78 °C for 5 hours in inert atmosphere. After cooling to 20 °C it is extracted with 100 ml of water. The aqueous phase is extracted with 30 ml of toluene, the unified toluene solution is extracted with 2 x 50 ml of 0.5 N NaOH-solution and 50 ml of water, dried over anhydrous Na₂SO₄, and stirred with activated carbon.
The toluene is evaporated in vacuo, the residue is solved in 100 ml of diethyl ether and 11.6 g (0.1 mole) of maleic acid is added. The obtained suspension is stirred for 5 hours, filtered, washed with diethyl ether and dried to give 25.2 g. of the title compound.
Melting point is 78-79 °C, HPLC purity is 97%.

### d) The synthesis of 4-[2-(5-ethyl-2-pyridinyl]ethoxy]-benzaldehyde oxalate.

15.1 g (0.1 mole) of 5-ethyl-2-(2-hydroxy-ethyl)-pyridine is solved in 150 ml of toluene and 16.8 ml (0.12 mole) of triethyl-amine is added. The obtained solution is cooled to 0-5 °C and 11.84 g (0.1 mole) of methanesulfonyl chloride is added dropwise in 30 minutes. The reaction mixture is stirred at 20 °C for 4 hours and then it is extracted with 100 ml of water. The aqueous phase is extracted with 20 ml of toluene, the unified toluene solution is dried over anhydrous Na₂SO₄.
To the filtered solution 70 ml of ethanol, 17.3 g (0.14 mole) of 4-hydroxy-benzaldehyde, 15 g (0.14 mole) of K₂CO₃ is added and it is stirred at 75-78 °C for 5 hours in an inert atmosphere. After cooling to 20 °C it is extracted with 100 ml of water. The aqueous phase is extracted with 30 ml of toluene, the unified toluene solution is extracted with 2 x 50 ml of 0.5 N NaOH-solution and 50 ml of water, dried over anhydrous Na₂SO₄, and stirred with activated carbon.
The toluene is evaporated in vacuo, the residue is solved in 150 ml of acetone and 10 g (0.08 mole) of oxalic acid dihydrate is added. The product is precipitated at once as a crystal. It is stirred at 20°C for 5 hours, and at 0 °C for 2 hours, filtered, washed with 0 °C acetone and dried at 50 °C to give 25.5 g (74 %) of the title compound.
Melting point is 120-122 °C, HPLC purity is 99.7%.

### Example 2

The synthesis of 5-[[4-[2-(5-ethyl-2-pyridinyl)ethoxy]phenyl]methylene]-thiazolidine-2,4-dione hydrogen chloride
15 g (43.4 mmole) of the product obtained in example 1.d) is suspended in 100 ml of ethyl acetate and it is extracted with 90 ml of 10 % K₂CO₃-solution. The aqueous phase is extracted with 2 x 50 ml of ethyl acetate, dried over anhydrous Na₂SO₄, and the ethyl acetate is evaporated in vauo..
The residue is solved in 150 ml of ethanol, 6 g (51 mmole) of thiazolidine-2,4-dione and 0.9 ml of piperidine is added and refluxed for 5 hours (e.g. after 1.5-2 hours the crystallization of the product is started).
The reaction mixture is diluted with 70 ml of ethanol and at 50-60 °C 19 ml of 3N hydrochloric acid solution is added. It is stirred for 1 hours, cooled to 5 °C and stirred again for 2 hours, filtered, washed with ethanol and dried at 60 °C to give 13.7 g (81 %) of the title compound.
This crude product is recrystallized from 180 ml of acetic acid (90,5 %).
Melting point is: 232-234 °C, HPLC purity is: 99,6%
¹-NMR:
1,24 (t, *J*=7,5 Hz, 3H, -CH₂C*H*₃); 2,79 (q, *J*=7,5 Hz, 2H, -C*H*₂CH₃); 3,53 (t, *J*=6,0 Hz, 2H, -OCH₂C*H*₂-); 4,52 (t, *J*=6,0 Hz, 2H, -OC*H*₂CH₂-); 7,06-7,14 (m, 2H, Ar*H*); 7,52-7,58 (m, 2H, Ar*H*); 7,74 (s, 1H, -C*H*=); 7,97 (d, *J*=8,1 Hz, 1H, Pyr-3); 8,40 (dd, *J*=8,1 Hz és 2,1 Hz, 1H, Pyr-4); 8,72 (d, *J*=2,1 Hz, 1H, Pyr-6); 12,50 (br s, 1H, HCl)

### Example 3

The synthesis of 5-[4-[2-(5-ethyl-2-pyridinyl)-ethoxy]phenyl]methyl]-2,4-thiazolidinedione-hydrogen chloride

9.77 g (25 mmole) of the product obtained in example 2 is solved in the mixture of 220 ml of methanol and 30 ml of water at 60 °C and it is hydrogenated in the presence of 1.5 g of 10 % palladium on carbon catalyst at 50-60 °C and on 5-6 bar pressure.
After approx. 15 hours of reaction time the catalyst is filtered and the solvent is evaporated in vacuo. The residue is solved in 100 ml of 1 N hydrochloric acid solution, and with stirring the product is precipitated as a crystal. The reaction mixture is stirred for 3 hours at 0-5 °C, filtered, washed with 20 ml of 0 °C ethanol, and it is dried in vacuum until permanent weight to give 8.25 g (84 %) of the title compound.

Melting point is: 196-198 °C, HPLC purity is: 99,7 %.

## Claims

1. 4-[2-(5-ethyl-2-pyridinyl)-ethoxy]-benzaldehyde salts of formula (II), 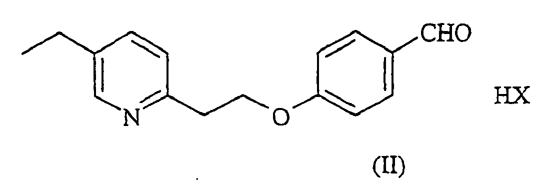 wherein HX is: HCl, CF₃COOH, C₄H₄O₄, (COOH)₂.

2. 4-[2-(5-ethyl-2-pyridinyl)-ethoxy]-benzaldehyde oxalate salt.

3. 5-[[4-[2-(5-ethyl-2-pyridinyl)ethoxy]phenyl]methylene]-2,4-thiazolidinedione hydrogen chloride of formula (III):

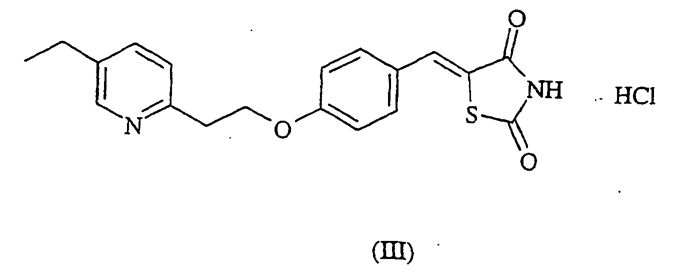

4. Process for the synthesis of pioglitazone hydrogen chloride of formula (I) 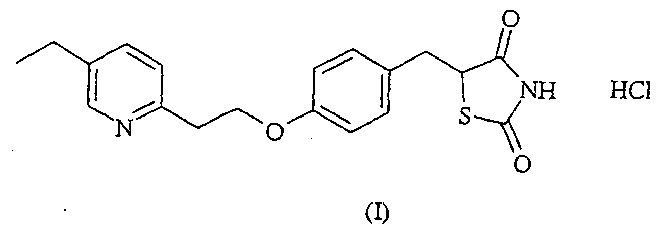 (chemical name of which is: (R,S)-5-[[4-[2-(5-ethyl-2-pyridinyl)ethoxy]phenyl] methyl]-2,4-tiazolidinedione hydrogen chloride) **characterized by** that
a) The 4-[2-(5-ethyl-2-pyridinyl)-ethoxy]-benzaldehyde salt of formula (II) 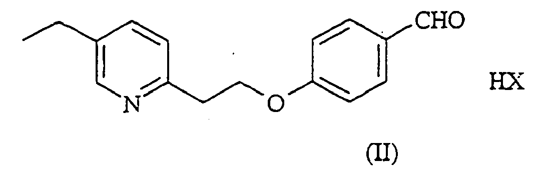 is reacted with a base in a desired case, then with thiazolidine-2,4-dione and the obtained 5-[[4-[2-(5-ethyl-2-pyridinyl)ethoxy]phenyl]methylene]-2,4-thiazolidine-dione base is treated with hydrochloric acid and
b) the obtained 5-[[4-[2-(5-ethyl-2-pyridinyl)ethoxy]phenyl]methylene]-2,4-thiazolidinedione hydrogen chloride of formula (III) is hydrogenated.

5. The process according to claim 4, **characterized by** that from 4-[2-(5-ethyl-2-pyridinyl)-ethoxy]-benzaldehyde salts of formula (II) the 4-[2-(5-ethyl-2-pyridinyl)-ethoxy]-benzaldehyde oxalate salt is used.

6. The process according to claim 4, **characterized by** that 5-[[4-[2-(5-ethyl-2-pyridinyl)ethoxy]phenyl]methylene]-2,4-thiazolidinedione hydrogen chloride of formula (III) is hydrogenated in the presence of a catalyst.

7. The process according to claim 6, wherein the catalyst is represented by palladium on carbon.

## Patentansprüche

1. 4-[2-(5-Ethyl-2-pyridinyl)ethoxy]benzaldehydsalze der Formel (II): 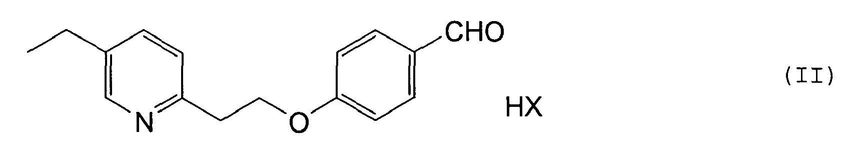 worin HX ist: HCl, CF₃COOH, C₄H₄O₄, (COOH)₂.

2. 4-[2-(5-Ethyl-2-pyridinyl)ethoxy]benzaldehydoxalatsalz.

3. 5-[[4-[2-(5-Ethyl-2-pyridinyl)ethoxy]phenyl]methylen]-2,4-thiazolidindionhydrogenchlorid der Formel (III):

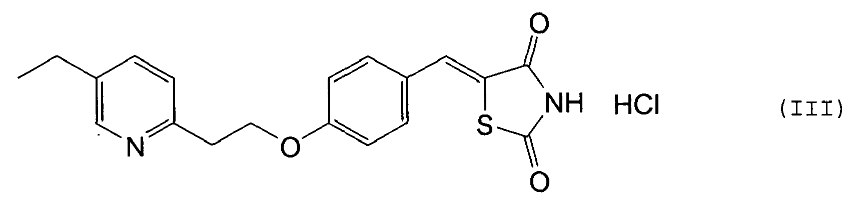

4. Verfahren zur Synthese von Pioglitazonhydrogenchlorid der Formel (I): 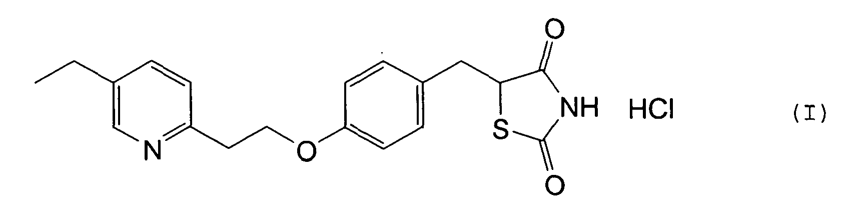 (dessen chemischer Name ist: (R,S)-5-[[4-[2-(5-Ethyl-2-pyridinyl)ethoxy]phenyl]methyl]-2,4-thiazolidindionhydrogenchlorid), **dadurch gekennzeichnet, daß**
a) das 4-[2-(5-Ethyl-2-pyridinyl)ethoxy]benzaldehydsalz der Formel (II): 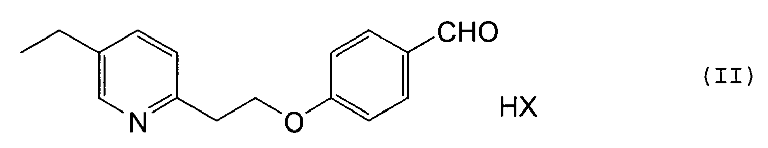 wenn gewünscht mit einer Base, dann mit Thiazolidin-2,4-dion umgesetzt wird, und das erhaltene 5-[[4-[2-(5-Ethyl-2-pyridinyl)ethoxy]phenyl]methylen]-2,4-thiazolidindion mit Salzsäure behandelt wird, und
b) das erhaltene 5-[[4-[2-(5-Ethyl-2-pyridinyl)ethoxy]phenyl]methylen]-2,4-thiazolidindionhydrogenchlorid der Formel (III) hydriert wird.

5. Verfahren gemäß Anspruch 4, **dadurch gekennzeichnet, daß** aus dem 4-[2-(5-Ethyl-2-pyridinyl)ethoxy]benzaldehydsalzen der Formel (II) das 4-[2-(5-Ethyl-2-pyridinyl)ethoxy]benzaldehydoxalatsalz verwendet wird.

6. Verfahren gemäß Anspruch 4, **dadurch gekennzeichnet, daß** das 5-[[4-[2-(5-Ethyl-2-pyridinyl)ethoxy]phenyl]methylen]-2,4-thiazolidindionhydrogenchlorid der Formel (III) in Gegenwart eines Katalysators hydriert wird.

7. Verfahren gemäß Anspruch 6, wobei der Katalysator durch Palladium auf Kohle dargestellt wird.

## Revendications

1. Sels de 4-[2-(5-éthyl-2-pyridinyl)-éthoxy]-benzaldéhyde de formule (II), 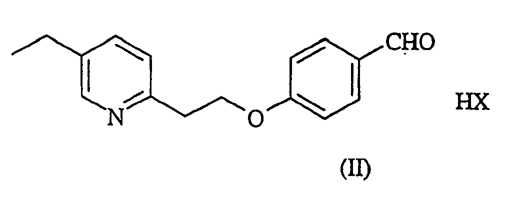 dans laquelle HX est : HCl, CF₃COOH, C₄H₄O₄, (COOH)₂.

2. Sel oxalate de 4-[2-(5-éthyl-2-pyridinyl)-éthoxy]-benzaldéhyde.

3. Chlorure d'hydrogène de 5-[[4-[2-(5-éthyl-2-pyridinyl)-éthoxy]phényl]méthylène]-2,4-thiazolidinedione de formule (III) :

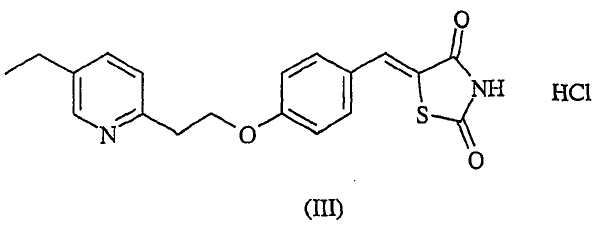

4. Procédé de synthèse de chlorure d'hydrogène de pioglitazone de formule (I) 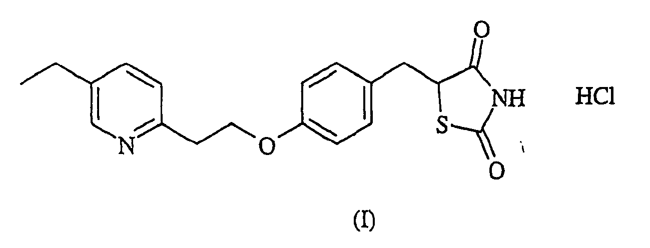 (dont le nom chimique est : chlorure d'hydrogène de (R,S)-5-[[4-[2-(5-éthyl-2-pyridinyl)-éthoxy]phényl]méthyl]-2,4-thiazolidinedione) **caractérisé en ce que** :
a) Le sel de 4-[2-(5-éthyl-2-pyridinyl)-éthoxy]-benzaldéhyde de formule (II) 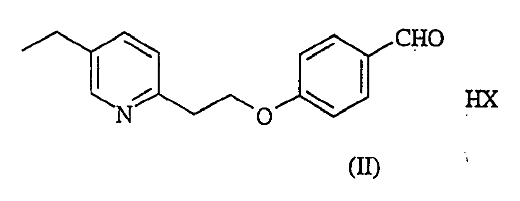 est mis à réagir avec une base dans un cas souhaité, puis avec la thiazolidine-2,4-dione et la base 5-[[4-[2-(5-éthyl-2-pyridinyl)-éthoxy]phényl]méthylène]-2,4-thiazolidinedione obtenue et traitée avec de l'acide chlorhydrique et
b) le chlorure d'hydrogène de 5-[[4-[2-(5-éthyl-2-pyridinyl)-éthoxy]phényl]méthylène]-2,4-thiazolidinedione obtenu de formule (III) est hydrogéné.

5. Procédé selon la revendication 4, **caractérisé en ce qu'**à partir de sels de 4-[2-(5-éthyl-2-pyridinyl)-éthoxy]-benzaldéhyde de formule (II), on utilise le sel oxalate de 4-[2-(5-éthyl-2-pyridinyl)-éthoxy]-benzaldéhyde.

6. Procédé selon la revendication 4, **caractérisé en ce que** le chlorure d'hydrogène de 5-[[4-[2-(5-éthyl-2-pyridinyl)-éthoxy]phényl]méthylène]-2,4-thiazolidinedione de formule (III) est hydrogéné en présence d'un catalyseur.

7. Procédé selon la revendication 6, dans lequel le catalyseur est représenté par du charbon palladié.
